# EUROPÄISCHE PATENTANMELDUNG

(11) **EP 1 084 714 A1**
(43) Veröffentlichungstag der Anmeldung: **21.03.2001**
(21) Anmeldenummer: 00118063.7
(22) Anmeldetag: 23.08.2000
(51) Int. Cl.: A61L 2/28, G01N 31/22

(54) **Sterilisationstestvorrichtung**

(30) Priorität: 18.09.1999 DE 19944847
(71) Anmelder: SECUNDUS Medizinische Kontrollsysteme GmbH, 65239 Hohenstein (DE)
(72) Erfinder: Schefter, Siegfried, 65329 Hohenstein (DE)
(74) Vertreter: von Creytz, Dietrich, Dipl.-Phys.

(57) **Zusammenfassung**

Es wird eine Sterilisationstestvorrichtung mit einer zur Aufnahme eines Indikators (3) vorgesehenen Testkammer (2) beschrieben. Die Vorrichtung soll es erlauben, beim Sterilisieren chirurgischer Gelenkgeräte, ohne die Geräte selbst zu kontrollieren, eine Aussage über das Maß der Sterilisation eines nur durch eine Spielpassung zugänglichen Gerätehohlraums zu machen. Die erfindungsgemäße Vorrichtung besitzt eine Zuleitung für eine Evakuierung und das Sterilisationsmittel. Der Entlüftungsweg dieser zur Testkammer führenden Zuleitung wird in Form einer Prüfpassung (9) ausgebildet, deren Durchlaßquerschnitt zumindest so eng wie die zu sterilisierende Spielpassung ist.

## Beschreibung

Die Erfindung betrifft eine Sterilisationstestvorrichtung mit einer zur Aufnahme eines Indikators vorgesehenen Testkammer, welche eine Zuleitung für eine Evakuierung sowie ein Einlassen des Sterilisationsmittels besitzt und im übrigen abgedichtet ist.

Derartige in einen gegebenenfalls das Sterilisationsgut aufnehmenden Sterilisator bzw. Sterilisationsraum zu setzende Testvorrichtungen bzw. Prüfkörper sollen es erlauben, bei dem jeweiligen Sterilisationsverfahren mit ausreichender Wahrscheinlichkeit anzuzeigen, ob die Sterilisation erfolgreich war. Als Sterilisationsmittel wird im Krankenhausbereich bzw. in der Medizintechnik bevorzugt eine gas- oder dampfförmige Sterilisationsatmosphäre, z.B. mit Wasserdampf, Ethylenoxid oder Formaldehyd, in den Sterilisator eingelassen. Bei der sogenannten Dampfsterilisation mit Sattdampf wird der Sterilisator vor dem Einlassen des Sattdampfes evakuiert. Dadurch wird erreicht, daß die ursprünglich im Sterilisationsraum befindliche Luft den Zugang des Sterilisationsmittels nicht behindern kann.

In DE 91 04 824 U1 wird ein Teströhrchen zum Einlegen eines chemischen Dampfsterilisations-Teststreifens beschrieben. Das Teströhrchen besteht aus zwei zylindrischen Hülsen, die an ihrer offenen Stirnfläche zusammensteckbar sind. Um das Teströhrchen so auszubilden, daß eine mehrmalige Verwendung behindert wird, soll es mit einem die beiden Hülsen an ihrer Verbindungslinie überlappenden Siegel beklebt werden.

In DE 197 24 155 A1 wird eine Sterilisationstestvorrichtung mit einer zur Aufnahme eines Hauptindikators vorgesehenen Testkammer beschrieben, die einerseits Zuleitungsmittel für das jeweilige Sterilisationsmittel und andererseits eine gegen ein Eindringen des Sterilisationsmittels mit Hilfe einer Kammerdichtung nach außen hin verschließbare Kontrollöffnung besitzt. Um zu erreichen, daß Fehler der Kammerdichtung bemerkt werden können, wird jenseits der Kammerdichtung außen an die Testkammer eine bei mangelhafter Kammerdichtung durch die Testkammer hindurch von dem Sterilisationsmittel zu flutende Hilfskammer angeschlossen, welche einen Hilfsindikator enthält.

In EP 0 628 814 A1 oder EP 0 657 177 B1 werden Sterilisationstestvorrichtungen beschrieben, welche in den Sterilisator zu geben sind. Die Sterilisation wird als erfolgreich angesehen, wenn ein in einer für das Sterilisationsmittel schwer erreichbarer Testkammer befindlicher Indikator verändert wurde und somit anzeigt, ob in der Testkammer ausreichende Sterilisationsbedingungen geherrscht haben oder nicht. Um den Sterilisator auf seine Aufgabe vorzubereiten, wird der ganze Sterilisationsraum zunächst evakuiert; erst anschließend wird das Sterilisationsmittel, z.B. Sattdampf für vier bis sechs Minuten bei einer Temperatur von 134 °C, in den Raum geleitet. Die einzige Verbindung zwischen Sterilisationsraum und Testkammer führt nach EP 0 628 814 A1 durch einen Schlauch, dessen Länge groß gegenüber seinem Durchmesser ist, oder nach EP 0 657 177 B1 durch einen Papierstöpsel.

Die Testkammer soll durch eine dünne rohrförmige Zuleitung bzw. durch den Papierstöpsel schwerer vollständig zu entlüften sein, als jeder Bereich des Innern der Sterilisationskammer (kurz Sterilisator) und der darin befindlichen Güter, die sterilisiert werden sollen. Die Testvorrichtung soll also so konstruiert werden, daß der in der Testkammer befindliche Indikator nur dann eindeutig verändert wird, wenn das Sterilisationsmittel sogar die Testkammer im wesentlichen ausfüllen kann. Das wiederum setzt eine vorherige praktisch vollständige Entlüftung der Testkammer voraus. Ein in der Testkammer durch das Sterilisationsmittel vorschriftsmäßig veränderter Indikator zeigt also an, daß der Sterilisationsraum vor dem Einlassen des Sterilisationsmittels, z.B. des Sattdampfes, evakuiert war.

Es gibt im Krankenhausbereich Geräte ― z.B. mit Gelenken, Gewinden oder engen Hohlräumen ―, deren gegeneinander bewegliche Teile als sogenannte Paßteile eine enge Spielpassung besitzen. Das gilt beispielsweise für chirurgische Scheren oder Klemmen. Diese Geräte weisen Scharniere mit Hohlräumen am Scharniergelenk bzw. an dessen Lagerwelle aber auch Verbindungsschrauben oder Nieten auf. Das Spiel der Paßteile an diesen Gelenkstellen usw. soll so klein wie möglich für eine gute Gängigkeit sein. Die Passungen können als Labyrinthdichtungen ausgebildet sein, um das Eindringen von Keimen zu erschweren. Die vorliegende Erfindung geht aus von der Erkenntnis, daß solche Geräte, welche selbst als sehr enge, aber gängige Spielpassungen ausgebildete Zugänge zu Hohlräumen aufweisen, nach der herkömmlichen Sterilisationsmethode eigentlich nur dann bis in ihr Innerstes sterilisiert werden können, wenn man entweder einen Indikator in den jeweiligen Geräte-Hohlraum selbst einbringt oder das Gerät während der Sterilisation demontiert. Ohne Umkonstruktion der Geräte ist das nicht möglich. Zum Schutz gegen das Eindringen von Keinem sind die fraglichen Hohlräume oft nur einseitig offen, das heißt, es handelt sich um endständige Hohlräume. ― Die Bedeutung des Begriffs 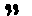 Passung" ist normiert; es gibt ISO-Passungen; vgl. Dubbel, Taschenbuch für den Maschinenbau, Band 1, Springer-Verlag 1974, Seiten 626 bis 630.

Der Erfindung liegt die Aufgabe zugrunde, eine Sterilisationstestvorrichtung zu schaffen, die es erlaubt, beim Sterilisieren der Spielpassungshohlräume, z.B. der beschriebenen chirurgischen Gelenkgeräte, ohne die in den Sterilisator gesetzten Geräte selbst zu kontrollieren, eine Aussage über das Maß der Sterilisation eines nur durch eine gerade noch gängige Spielpassung erreichbaren Gerätehohlraums zu machen.

Die erfindungsgemäße Lösung wird im Anspruch 1 angegeben. Einige Verbesserungen und weitere Ausgestaltungen der Erfindung werden in den Unteransprüchen beschrieben.

Erfindungsgemäß wird der Entlüftungsweg der im Sterilisator zur Testkammer führenden Zuleitung als Prüfpassung ausgebildet. Vorzugsweise besitzen die Prüfpaßteile des Entlüftungswegs der zur Testkammer führenden Zuleitung ein Paßmaß, welches zumindest so eng wie die Spielpassung des Zugangs eines zu sterilisierenden Geräte-Hohlraums ist. Mit anderen Worten heißt das, daß der Weg von der Umgebung der Testvorrichtung (innerhalb des Sterilisators) in die Testkammer hinein durch einen Spalt (oder dergleichen Grenze zwischen zwei Paßteilen) mit extrem engem Durchlaßquerschnitt führen soll. Auch dieser Spalt kann im vorliegenden Zusammenhang als Prüfpassung bezeichnet werden. Das Größtmaß bzw. Größtspiel der Prüfpassung soll vorzugsweise höchstens 0,1 mm betragen. Die Prüfpassung soll zwischen zwei, bevorzugt zylinderförmig, ineinander geführten Paßteilen vorgesehen werden.

Nach Vorstehendem der Weg der beim Evakuieren aus der Testkammer abgesaugten Luft durch eine Prüfpassung, welche die zu kontrollierende Geräte-Spielpassung simuliert. Vorzugsweise soll der Indikator in einer einseitig offenen bzw. endständigen Testkammer positioniert werden. Das gilt insbesondere dann, wenn der zu prüfende Hohlraum endständig ist, das heißt nur einen Eingang ― also keinen Durchgang ― besitzt.

Bei den zu sterilisierenden Geräten mit gängig gegeneinander beweglichen Teilen kann es sich ― wie gesagt ― um chirurgische Arbeitsmittel, wie Scheren oder Klemmen, handeln. Die gegeneinander beweglichen Messer, Hebel usw. dieser Geräte werden an einer Scharnierachse gängig ― das heißt mit Spiel ― miteinander verbunden. An der Scharnierachse ist nämlich ein gewisses Spiel erforderlich, um das Gerät bestimmungsgemäß benutzen zu können.

Das Simulieren der zu sterilisierenden, beispielhaft beschriebenen Spielpassung erfordert erfindungsgemäß einen nur durch eine Prüfpassung hindurch zu evakuierenden Hohlraum mit Indikator. Dieser Hohlraum kann endständig angeordnet und zum besseren Schutz gegen das Eindringen von Keimen labyrinthartig zugänglich bzw. ausgebildet sein. Demgemäß wird gemäß weiterer Erfindung vorgesehen, die die tatsächliche Spielpassung simulierende Zuleitung als quer zum Eingang einer als Sackloch ausgebildeten Testkammer stehende Prüfpassung auszubilden. Auf diese Weise läßt sich ausgezeichnet indirekt kontrollieren, ob im Sterilisator ein (anderer) endständiger Geräte-Hohlraum keimfrei gemacht worden ist.

Die erfindungsgemäß Sterilisationstestvorrichtung erlaubt es, beim Sterilisieren chirurgischer Gelenkgeräte, ohne die Geräte selbst zu kontrollieren, eine Aussage über das Maß der Sterilisation eines nur durch eine Spielpassung zugänglichen Gerätehohlraums zu machen. Die Testvorrichtung besitzt eine Zuleitung für eine Evakuierung und das Sterilisationsmittel. Der Entlüftungsweg dieser zur Testkammer führenden Zuleitung wird in Form einer Prüfpassung ausgebildet, deren Durchlaßquerschnitt zumindest so eng wie die zu sterilisierende Spielpassung ist.

Anhand der schematischen Darstellung eines Ausführungsbeispiels werden Einzelheiten der Erfindung erläutert.

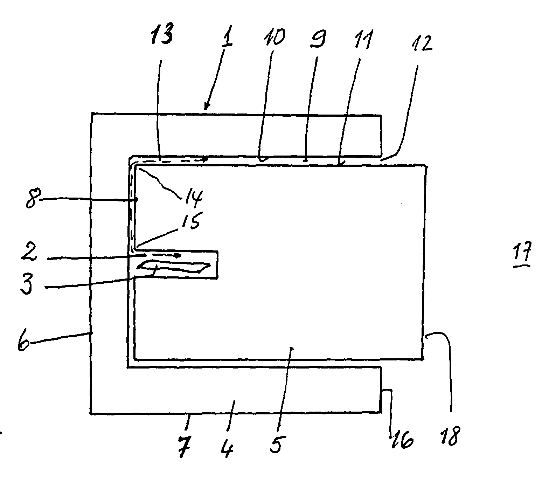

Die beiliegende Zeichnung zeigt einen Schnitt durch einen insgesamt mit 1 bezeichneten Prüfkörper, der in einen Sterilisator zu legen ist. Der ― wegen der bequemen Handhabbarkeit ― innen bevorzugt zylindrische Prüfkörper 1 enthält eine Testkammer 2 mit darin befindlichem Indikator 3. Die Testkammer 2 befindet sich in einer Prüfkammer 4, in welche ein (zylindrischer) Kern 5 passend einzustecken ist. Beispielsweise wird die Prüfkammer 4 becherförmig ― wie dargestellt ― mit Boden 6 und Wandung 7 ausgebildet. Gegebenenfalls soll der Kern 5 als Zylinder ― mit nur geringem Spiel passend zum Innenraum der Prüfkammer 4 ― geformt werden. Der Kern 5 enthält einen Hohlraum, nämlich die Testkammer 2. Wegen der Zylinderform kann der Kern 5 leicht in die Kammer 4 gesteckt oder aus der Kammer 4 herausgezogen werden.

Im Ausführungsbeispiel enthält der Kern 5 die Testkammer 2 in seinem Boden 8, welcher dem Boden 6 der Prüfkammer 4 zuzuwenden ist. Die Verbindung zwischen dem Außenraum des Prüfkörpers 1 und der Testkammer 2 wird erfindungsgemäß durch eine (enge) mechanische Prüfpassung 9 gebildet. Im Ausführungsbeispiel befindet sich die Prüfpassung 9 zwischen der Innenwandung 10 der Prüfkammer 4 und der Außenwandung 11 des Kerns 5. Die Testkammer 2 kann also nur durch die Prüfpassung 9 bzw. deren (im Ausführungsbeispiel) ringförmigen Eingang 12 entlüftet bzw. bedämpft werden.

Im Ausführungsbeispiel enthält die Prüfpassung 9 zwei Umlenkungen des Verbindungswegs 13 zwischen Eingang 12 und Kammer 2, nämlich an den gezeichneten Kanten 14 und 15. Der Kern 5 steht im Ausführungsbeispiel etwas über den Rand 16 der Prüfkammer 4 über, um ein Herausziehen des Kerns 5 im Anschluß an einen Test ― zwecks Kontrolle des Indikators 3 ― zu erleichtern. Die Kanten 14, 15 können eine labyrinthartige Spielpassung des zu sterilisierenden Geräts simulieren.

Wichtig im Rahmen der Erfindung ist vor allem die Prüfpassung 9 zwischen dem den Prüfkörper 1 umgebenden Außenraum 17 (im Sterilisator) und dem Innern der Testkammer 2. In diesem Zusammenhang ist die Form des Prüfkörpers 1 bzw. von Prüfkammer 4 und Kern 5 nicht wesentlich. Beispielsweise kann anstelle der gezeichneten Zylinder- bzw. Rohrform auch eine Form mit in Umfangsrichtung kantiger Prüfpassung vorgesehen werden. Es ist auch nicht entscheidend, daß die Testkammer 2 dem Boden 6 eines Bechers zugewendet ist. Im Prinzip kann die Testkammer 2 auch an einer Umfangswandung des Kerns vorgesehen werden. Der Kern 5 kann voll ― wie gezeichnet ― aber auch im wesentlichen hohl sein.

Weiterhin liegt es im Rahmen der Erfindung, den ringförmigen Eingang 12 der Prüfpassung 9 als enges Loch an dem dem Boden 8 gegenüberliegenden Kopf 18 des Kerns 5 auszubilden. In diesem Fall kann es, für eine Demontage zwecks Prüfung des Untersuchungsergebnisses am Indikator 3 sinnvoll sein, den Boden 6 der Prüfkammer 4 abnehmbar (von der Wandung 7) auszubilden. Ein Beispiel einer solchen Prüfkammer wird beschrieben in DE 197 24 155 A1.

Das Spiel einer im Sinne der ISO-Normen gängigen Spielpassung zwischen beweglichen Teilen eines chirurgischen Instruments kann eine Größe von weniger als 0,1 mm haben. Eine entsprechend enge ― bevorzugt noch engere ― Prüfpassung 9 soll in dem erfindungsgemäßen Prüfkörper als Verbindungsweg 13 zwischen Außenraum 17 und Testkammer 2 vorgesehen werden. Die Prüfpassung 9 soll also so eng sein, daß die Testkammer 2 (durch die Prüfpassung hindurch) schwieriger zu evakuieren ist, als die eigentlich zu sterilisierende Spielpassung.

### Bezugszeichenliste

- 1: = Prüfkörper
- 2: = Testkammer
- 3: = Indikator
- 4: = Prüfkammer
- 5: = Kern
- 6: = Boden (4)
- 7: = Wandung
- 8: = Boden (5)
- 9: = Prüfpassung
- 10: = Innenwandung (4)
- 11: = Wandung (5)
- 12: = Eingang
- 13: = Weg
- 14: = Kante
- 15: = Kante
- 16: = Rand
- 17: = Außenraum
- 18: = Kopf (5)

## Patentansprüche

1. Sterilisationstestvorrichtung mit einer zur Aufnahme eines Indikators (3) vorgesehenen Testkammer (2), welche eine Zuleitung für eine Evakuierung und ein Einlassen des Sterilisationsmittels besitzt und im übrigen abgedichtet ist, **dadurch gekennzeichnet**, daß der Entlüftungsweg (13) der zur Testkammer (2) führenden Zuleitung als durch den durch das Spiel zwischen Paßteilen (4,5) bedingter Spalt definierte Prüfpassung (9) ausgebildet ist.

2. Vorrichtung nach Anspruch 1, **dadurch gekennzeichnet**, daß das Spiel der, vorzugsweise zwischen Paßteilen (4, 5) aufgespannten, Prüfpassung (9) zumindest so eng wie dasjenige der zu sterilisierenden Spielpassung eines jeweils zusammen mit der Testvorrichtung in einen Sterilisator zu legenden Gutes ist.

3. Vorrichtung nach Anspruch 1 oder 2, **dadurch gekennzeichnet**, daß das Größtmaß bzw. Größtspiel der Prüfpassung höchstens 0,1 mm beträgt.

4. Vorrichtung nach mindestens einem der Ansprüche 1 bis 3, **dadurch gekennzeichnet**, daß der Weg (13) durch die zu der Testkammer führende Prüfpassung (9) im wesentlichen quer zum Testkammer-Eingang gerichtet ist.

5. Vorrichtung nach Anspruch 4, **dadurch gekennzeichnet**, daß der Weg (13) zwischen Eingang (12) der Prüfpassung (9) und Eingang der Testkammer (2) um mindestens eine etwa rechtwinklige Kante (14, 15) führt.

6. Vorrichtung nach mindestens einem der Ansprüche 1 bis 5, **dadurch gekennzeichnet**, daß die Prüfpassung (9) zwischen zwei, bevorzugt zylinderförmig, ineinandergefügten Paßteilen (4, 5) vorgesehen ist.
